(19) Europäisches Patentamt
European Patent Office
Office européen des brevets

(11) Numéro de publication: **0 418 933 A1**

# (12) DEMANDE DE BREVET EUROPEEN

(21) Numéro de dépôt: **90121043.5**

(22) Date de dépôt: **26.09.88**

(51) Int. Cl.[5]: **C07C 323/51**, C07C 229/34, C07C 69/00, A61K 31/10

Cette demande a été déposée le 02 - 11 - 1990 comme demande divisionnaire de la demande mentionnée sous le code INID 60.

(30) Priorité: **28.09.87 FR 8713357**

(43) Date de publication de la demande: **27.03.91 Bulletin 91/13**

(60) Numéro de publication de la demande initiale en application de l'article 76 CBE : **0 310 484**

(84) Etats contractants désignés: **AT BE CH DE ES FR GB GR IT LI LU NL SE**

(71) Demandeur: **RHONE-POULENC SANTE**
**20, avenue Raymond Aron**
**F-92160 Antony(FR)**

(72) Inventeur: **Malleron, Jean-Luc**
**2 allée Renoir**
**F-91460 Marcoussis(FR)**
Inventeur: **Ponsinet, Gérard**
**7 rue de Grand Champ**
**F-94370 Sucy en Brie(FR)**
Inventeur: **Roussel, Gérard**
**20 ter rue des Carrières**
**F-91450 Soisy sur Seine(FR)**

(74) Mandataire: **Savina, Jacques et al**
**RHONE-POULENC SANTE, Service Brevets,**
**20 Avenue Raymond Aron**
**F-92165 Antony Cédex(FR)**

## (54) Dérivés d'alcadiènes, leurs préparations, les médicaments les contenant et produits intermédiaires.

(57) Composés de formule :

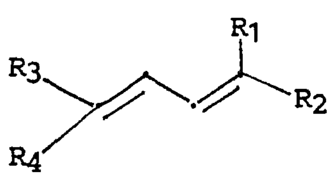

(I)

dans laquelle $R_1$ = hydroxy ou acétoxy, $R_2$ = hydrogène, carboxy, alcoxycarbonyle, phényle ou benzoyle et
- soit $R_3$ = alkylthio ou alcoxy et $R_4$ = naphtoyle ou benzoyle éventuellement substitué
- soit $R_3$ et $R_4$ forment avec l'atome de carbone auquel ils sont rattachés un cycle

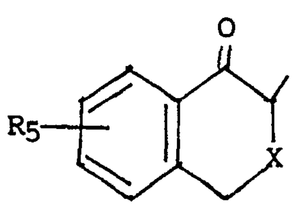

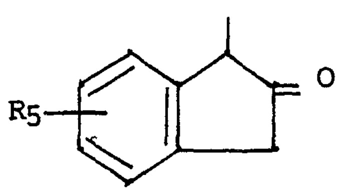

dans lesquelles $R_5$ = hydrogène ou alcoxy et X - méthylène ou S.

Procédés de préparation de ces composés, produits intermédiaires pour leur préparation et médicaments

EP 0 418 933 A1

les contenant.

# DERIVES D'ALCADIENES, LEURS PREPARATIONS, LES MEDICAMENTS LES CONTENANT ET PRODUITS INTERMEDIAIRES

La présente invention concerne des dérivés de formule :

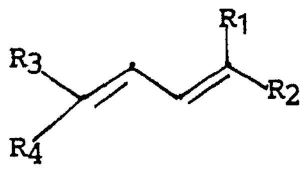

(I)

leurs procédés de préparation, les médicaments les contenant et les produits intermédiaires utiles à leur préparation.

Dans la formule (I), $R_1$ représente un radical hydroxy ou acétoxy, $R_2$ représente un atome d'hydrogène, un radical carboxy, alcoxycarbonyle, phényle ou benzoyle et

- soit $R_3$ représente un radical alkylthio ou alcoxy et $R_4$ représente un radical naphtoyle, benzoyle ou benzoyle substitué par un ou plusieurs atomes d'halogène, un ou plusieurs radicaux alkyle, alcoxy, phényle, phénoxy, pipéridino, diméthylamino ou hydroxy ou en positions -3 et -4 par un radical isopropylènedioxy,
- soit $R_3$ et $R_4$ forment ensemble avec l'atome de carbone auquel ils sont rattachés un des cycles de formules :

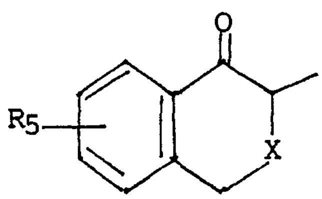

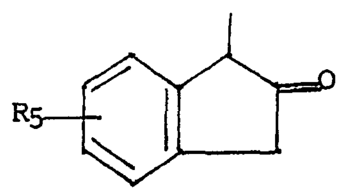

dans lesquelles $R_5$ représente un atome d'hydrogène ou un radical alcoxy et X représente un radical méthylène ou un atome de soufre, étant entendu que, lorsque $R_1$ représente un radical acétoxy, $R_4$ ne peut pas représenter un radical benzoyle substitué par un ou plusieurs radicaux hydroxy, que, sauf mention contraire, dans les définitions qui précèdent et celles qui seront citées ci-après, les radicaux alkyle et alcoxy, et les portions alkyle et alcoxy contiennent 1 à 4 atomes de carbone en chaîne droite ou ramifiée et que l'invention concerne également les formes tautomères des produits cités lorsque $R_1$ représente un radical hydroxy.

Selon l'invention, les produits de formule (I) dans laquelle $R_1$ représente le radical hydroxy, $R_2$ représente un atome d'hydrogène ou un radical alxocycarbonyle, phényle ou benzoyle et les autres symboles sont définis comme précédemment peuvent être préparés par hydrolyse acide des énamines de formule :

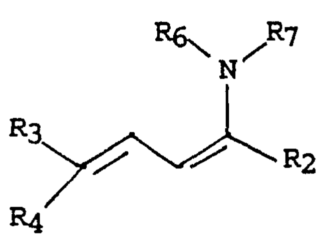

(II)

dans laquelle $R_2$ représente un atome d'hydrogène ou un radical alcoxycarbonyle, phényle ou benzoyle, $R_6$ et $R_7$ représentent des radicaux alkyle ou $R_6$ et $R_7$ forment ensemble avec l'atome d'azote auquel ils sont rattachés un hétérocycle tel que le cycle pipéridine ou le cycle morpholine, $R_3$ et $R_4$ ont la même signification que dans la formule (I).

Il est préférable d'effectuer cette hydrolyse en présence de 1 à 5 équivalents d'un acide minéral 1N à

12N, éventuellement en présence d'un solvant miscible à l'eau, à une température variant de 20°C à 100°C. Comme acide minéral, on peut citer les acides chlorhydrique, bromhydrique et sulfurique et comme solvant, les alcools (méthanol, éthanol, propanol, isopropanol), le tétrahydrofuranne ou le dioxanne.

Ces énamines, sont nouvelles et font partie de l'invention.

Les énamines de formule (II) peuvent être préparées par action d'un sel de vinamidinium de formule :

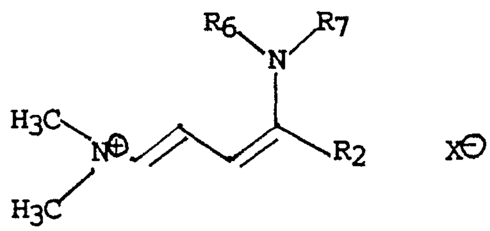

(III)

dans laquelle $R_2$, $R_6$ et $R_7$ ont la même signification que dans la formule (II) et X représente $BF_4$, Cl, $ClO_4$, Br, sur un composé à méthylène activé $R_3$-$CH_2$-$R_4$ pour lequel $R_3$ et $R_4$ ont la même signification que dans la formule (II).

Cette réaction s'effectue en présence d'une base parmi lesquelles on peut citer le méthylate, l'éthylate ou le tertiobutylate de sodium ou de potassium, dans l'alcool correspondant à une température comprise entre 20°C et la température d'ébullition du solvant. On peut également utiliser comme base un dérivé lithié tel que le méthyllithium, le butyllithium, le diisopropylamidure de lithium dans un solvant inerte tel que le tétrahydrofuranne ou l'oxyde de diéthyle à une température comprise entre -78°C et la température d'ébullition du solvant.

Les sels de vinamidinium de formule (III) peuvent être préparés par action d'une amine secondaire $HNR_6R_7$ pour laquelle $R_6$ et $R_7$ ont la même signification que dans la formule (II), sur les produits de formule :

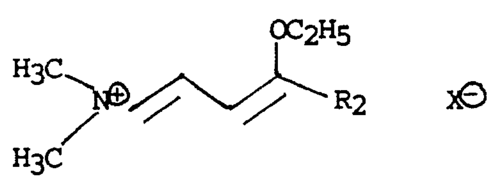

(IV)

dans laquelle $R_2$ et X ont la même signification que dans la formule (III).

La réaction est effectuée de préférence dans un solvant chloré tel que le chloroforme, le chlorure de méthylène ou le dichloro-1,2 éthane, à une température comprise entre 0°C et la température d'ébullition du solvant.

Les composés de formule (IV) peuvent être obtenus par application ou adaptation de la méthode décrite par R. GOMPPER et coll., Angew. Chem. Int. Ed. Eng., 17, 760-3 (1978). Cette méthode consiste à faire réagir un sel d'alkyloxonium comme le tétrafluoroborate de triéthyloxonium, sur une énamine de formule :

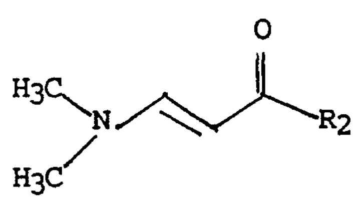

(V)

dans laquelle $R_2$ a la même signification que dans la formule (IV).

La réaction est, de préférence, effectuée dans un solvant chloré tel que le dichlorométhane ou le chloroforme, à une température comprise entre 20°C et la température d'ébullition du solvant.

Les composés de formule (V) peuvent être obtenus par application ou adaptation des méthodes décrites par R.F. ABDULLA et coll. Tetrahedron 35,1675-1734 (1979). Ces méthodes consistent à faire réagir un dialkylacétal de N,N-diméthylformamide avec un composé $CH_3$-CO-$R_2$ pour lequel $R_2$ a la même

signification que dans la formule (V).

De nombreux composés de formule $R_3$-$CH_2$-$R_4$ sont connus. Parmi ceux-ci, on peut citer l'α-méthoxyacétophénone (R.B. MOFFET et al, Org. Synth., 3, 562, 1955), l'α-tétralone (Beil 7, 370), le méthoxy-6 oxo-1 tétrahydro-1,2,3,4 naphtalène (Beil 9(2), 889), l'indanone-2 (Beil 7, 363).

Les composés de formule $R_3$-$CH_2$-$R_4$ nouveaux peuvent être obtenus par application ou adaptation des méthodes décrites pour les composés connus et des méthodes décrites ci-dessous et dans les exemples.

Les composés de formule $R_3$-$CH_2$-$R_4$ dans laquelle $R_3$ représente un radical alkylthio et $R_4$ représente un radical naphtoyle, benzoyle ou benzoyle substitué par un ou plusieurs atomes d'halogène, un ou plusieurs radicaux alkyle, alcoxy, phényle, phénoxy, hydroxy, pipéridino, diméthylamino ou en positions -3 et -4 par un radical isopropylènedioxy peuvent être préparés par action des composés $R_{11}$-SNa pour lesquels $R_{11}$ représente un radical alkyle, sur un halogénure de formule $R_4$-$CH_2$-X dans laquelle $R_4$ a la même signification que précédemment et X représente un atome d'halogène. Cette réaction s'effectue de préférence dans un alcool tel que le méthanol ou l'éthanol à une température comprise entre 20° C et 60° C.

Le chlorure de formule $R_4$-$CH_2Cl$ dans laquelle $R_4$ représente un radical benzoyle substitué par un radical pipéridine peut être préparé par action de chlorure de titane III sur le dérivé dibromé $R_4$-CO-CH-$Br_2$ pour lequel $R_4$ a la même signification que précédemment, en présence d'un acide minéral tel que l'acide chlorhydrique à une température d'environ 100° C.

Le composé de formule $R_4$-CO-CH-$Br_2$ peut être obtenu par action d'un mélange brome-acide bromhydrique sur le dérivé cétonique correspondant, de préférence à 20° C.

Les autres nouveaux halogénures de formule $R_4$-$CH_2$-X peuvent être préparés par halogénation des composés $R_4$-$CH_3$ correspondants par des méthodes connues en soi telles que celles décrites par MARCH, Advanced Organic Chemistry, 1977, p. 537-39 (second edition) ou par adaptation des méthodes de préparation des halogénures connus.

Les composés $R_{11}$-SNa peuvent être obtenus par action d'une base telle que le méthylate ou l'éthylate de sodium sur le thiol correspondant.

Les composés de formule $R_3$-$CH_2$-$R_4$ dans laquelle $R_3$ représente un radical alcoxy et $R_4$ représente un radical naphtoyle, benzoyle ou benzoyle substitué par un ou plusieurs atomes d'halogène ou un ou plusieurs radicaux phényle, phénoxy, hydroxy, pipéridino, diméthylamino, ou en positions -3 et -4 par un radical isopropylènedioxy peuvent être obtenus par application ou adaptation de la méthode décrite par R.B. MOFFET et al., Org. Synth., 3, 562 (1955).

Les composés de formule :

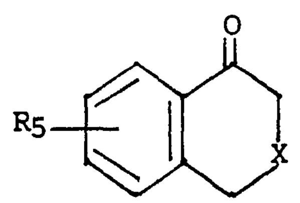

dans laquelle $R_5$ représente un atome d'hydrogène ou un radical alcoxy et X représente un atome de soufre peuvent être obtenus en chauffant l'acide benzylthioacétique éventuellement substitué par un radical alcoxy dans un solvant tel que le nitrobenzène, en présence d'un acide tel que l'acide polyphosphorique à une température comprise entre 50° C et 180° C.

Selon l'invention, les produits de formule (I) dans laquelle $R_1$ représente le radical hydroxy, $R_2$ représente un radical alcoxycarbonyle et les autres symboles sont définis comme précédemment peuvent également être préparés par hydrolyse acide des énamines de formule :

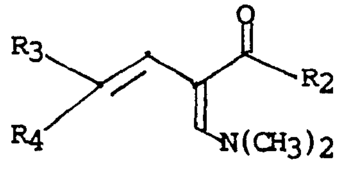

(VI)

dans laquelle $R_2$ représente un radical alcoxycarbonyle, $R_3$ et $R_4$ ont la même signification que dans la formule (I).

Cette hydrolyse peut s'effectuer dans les mêmes conditions opératoires que l'hydrolyse des énamines de formule (II).

Les énamines de formule (VI) peuvent être obtenues par action d'un dialkylacétal de N,N-diméthylformamide sur un acide correspondant de formule (I) dans laquelle $R_2$ représente le radical carboxy, $R_1$ représente le radical hydroxy, $R_3$ et $R_4$ sont définis comme précédemment. On opère de préférence dans un solvant chloré tel que le chloroforme ou le dichlorométhane à une température voisine de 20 °C.

Les énamines de formule (VI) sont nouvelles et font partie de l'invention.

Selon l'invention, les produits de formule (I) dans laquelle $R_1$ représente un radical hydroxy, $R_2$ représente le radical carboxy et les autres symboles sont définis comme précédemment, peuvent être préparés par saponification de l'ester énol correspondant de formule :

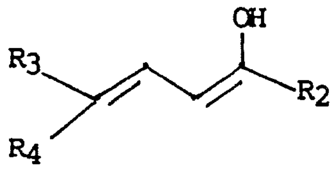

(VII)

dans laquelle $R_2$ représente un radical alcoxycarbonyle et $R_3$, $R_4$ sont définis comme précédemment.

De préférence, cette réaction s'effectue au moyen de 2 à 10 équivalents de soude ou de potasse 1N à 4N éventuellement en présence d'un solvant inerte miscible à l'eau tel que le méthanol, l'éthanol, le propanol, l'isopropanol ou le tétrahydrofuranne, à une température comprise entre 20 °C et 50 °C.

Selon l'invention, les produits de formule (I) dans laquelle $R_1$ représente un radical acétoxy et les autres symboles sont définis comme précédemment, étant entendu que $R_3$ ne peut pas représenter un radical benzoyle substitué par un ou plusieurs radicaux hydroxy, peuvent être obtenue par action du chlorure d'acétyle sur l'énol correspondant de formule (I) dans laquelle $R_1$ représente le radical hydroxy. Cette réaction s'effectue de préférence dans un solvant inerte tel que le tétrahydrofuranne en présence d'un accepteur d'acide tel que la triéthylamine à une température d'environ 20 °C.

Les mélanges réactionnels obtenus par les divers procédés décrits précédemment sont traités suivant des méthodes classiques physiques (évaporation, extraction, distillation, cristallisation, chromatographie) ou chimiques.

Les composés de formule (I) présentent despropriétés pharmacologiques intéressantes. Ces composés inhibent la 5-lipoxygénase et sont donc utiles comme antiinflammatoires, comme agents protecteurs notamment sur le tractus gastrointestinal et pour le traitement de l'asthme, des maladies allergiques, du psoriasis, de l'arthrite rhumatoïde et des fibroses notamment des fibroses hépathiques.

L'inhibition de la 5-lipoxygénase a été déterminée sur des cellules RBL-1 selon les méthodes de M.M. STEINHOFF et coll., B.B.B., 618, 28-34, 1980 et B.A. JAKSCHIK et coll., J. Biol. Chem., 257, 5346, 1982. Dans ce test, la $CI_{50}$(M) des composés de formule (I) est comprise entre $10^{-5}$ et $10^{-7}$.

Ces composés sont également actifs sur cellules PMN selon la méthode de H. SAFAYHI et coll., Biochem. Pharmacol., 34(15), 2691-4, 1985. Dans ce test, la $CI_{50}$(M) des composés de formule (I) est comprise entre $10^{-5}$ et $10^{-7}$.

Les produits de formule (I) présentent une faible toxicité. Leur $DL_{50}$ est supérieure à 100 mg/kg par voie orale chez la souris.

Sont particulièrement intéressants :

- les composés de formule (I) dans laquelle $R_1$ représente un radical hydroxy, $R_2$ représente un radical alcoxycarbonyle, $R_3$ représente un radical alkylthio et $R_4$ représente un radical benzoyle substitué par un atome d'halogène, un radical pipéridino, deux radicaux hydroxy ou en positions -3 et -4 par un radical isopropylènedioxy ;
- et les formes tautomères de ces produits lorsque $R_1$ représente un radical hydroxy.

Sont d'un intérêt particulier, les produits suivants :

- (chloro-4 phényl)-6 hydroxy-2 méthylthio-5 oxo-6 hexadiène-2,4 oate d'éthyle,
- hydroxy-2 méthylthio-5 oxo-6 (pipéridino-4 phényl)-6 hexadiène-2,4 oate d'éthyle,
- hydroxy-2 (isopropylènedioxy-3,4 phényl)-6 méthylthio-5 oxo-6 hexadiène-2,4 oate d'éthyle,
- hydroxy-2 méthoxy-5 oxo-6 phényl-6 hexadiène-2,4 oate d'éthyle,

Les exemples suivants, donnés à titre non limitatif, montrent comment l'invention peut être mise en pratique.

EXEMPLE 1 (exemple de référence)

A une solution de 60 g de diméthylamino-2 phénylthio-5 hexadiène-2,4 dioate de diéthyle dans 340 cm3 d'éthanol, on ajoute, en 10 minutes, en maintenant la température à environ 40 ° C, 344 cm3 d'une solution aqueuse d'acide chlorhydrique 1N. La suspension obtenue est refroidie à une température d'environ 20 ° C et est maintenue 30 minutes à cette température. Le précipité est filtré, lavé par 3 fois 50 cm3 d'eau et séché à 20 ° C sous pression réduite (20 mm de mercure ; 2,7 kPa) en présence d'anhydride phosphorique. On obtient 54 g d'un solide cristallisé. 13 g de ce solide sont recristallisés dans 30 cm3 d'oxyde d'isopropyle bouillant. On obtient ainsi 10 g d'hydroxy-2 phénylthio-5 hexadiène-2,4 dioate de diéthyle fondant à 98 ° C.

Le diméthylamino-2 phénylthio-5 hexadiène-2,4 dioate de diéthyle peut être obtenu de la façon suivante :

A une solution de 68,7 g phénylthioacétate d'éthyle et de 100 g de tétrafluoroborate de N-(diméthylamino-3 éthoxycarbonyl-3 propénylidène) N-méthyl méthanaminium dans 120 cm3 d'éthanol, on ajoute goutte à goutte, en 50 minutes et maintenant la température à environ 20 ° C, 210 cm3 d'une solution éthanolique 2M d'éthylate de sodium. Le mélange réactionnel est maintenu 2 heures 20 minutes à une température voisine de 20 ° C puis le solvant est distillé sous pression réduite (20 mm de mercure ; 2,7 kPa) à 40 ° C. Le résidu est repris par 1000 cm3 d'eau distillée et extrait par 3 fois 300 cm3 d'acétate d'éthyle. Après lavage à l'eau, les extraits organiques sont séchés sur sulfate de magnésium anhydre et concentrés à sec sous pression réduite (20 mm de mercure ; 2,7 kPa). Après recristallisation dans 150 cm3 d'oxyde d'isopropyle bouillant, on obtient 107 g d'un solide fondant à 70 ° C.

15 g de ce solide sont chromatographiés sur une colonne de 6 cm de diamètre contenant 450 g de silice avec un mélange de cyclohexane et d'acétate d'éthyle (80-20 en volumes) comme éluant. La première fraction de 800 cm3 est éliminée puis on recueille des fractions de 100 cm3. Les fractions 4 à 11 sont réunies et concentrées à sec à 40 ° C sous pression réduite (20 mm de mercure ; 2,7 kPa). Après recristallisation dans 40 cm3 d'oxyde d'isopropyle bouillant, on obtient 13,2 g de diméthylamino-2 phénylthio-5 hexadiène-2,4 dioate de diéthyle fondant à 76 ° C.

EXEMPLE 2 (exemple de référence)

Le tétrafluoroborate de N-(diméthylamino-3 éthoxycarbonyl-3 propénylidène) N-méthyl méthanaminium peut être obtenu de la façon suivante :

A une solution chlorométhylénique de 1007 g de tétrafluoroborate de N-(éthoxy-3 éthoxycarbonyl-3 propénylidène) N-méthyl méthanaminium, on ajoute, en 1 heure 20 minutes, à une température d'environ 20 ° C, une solution chlorométhylénique de 233 cm3 de diméthylamine. On coule ensuite lentement dans le milieu réactionnel 2000 cm3 d'éther éthylique anhydre puis sépare par filtration le produit cristallisé et le sèche sous pression réduite (20 mm de mercure ; 2,7 kPa) à une température d'environ 20 ° C. On obtient ainsi 925 g de tétrafluoroborate de N-(diméthylamino-3 éthoxycarbonyl-3 propénylidène) N-méthyl méthanaminium fondant à 102 ° C.

Le tétrafluoroborate de N-(éthoxy-3 éthoxycarbonyl-3 propénylidène) N-méthyl méthanaminium peut être obtenu de la façon suivante :

A une solution chlorométhylénique de 862 g de tétrafluoroborate de triéthyloxonium refroidie à 10 ° C et traversée par un courant d'argon, on ajoute, en 3 heures, 675 g de diméthylamino-4 oxo-2 butène-3 oate d'éthyle en maintenant la température à environ 10 ° C. Le milieu réactionnel est ensuite agité 5 heures à une température voisine de 20 ° C. La soluticn chlorométhylénique de tétrafluoroborate de N-(éthoxy-3 éthoxycarbonyl-3 propénylidène) N-méthyl méthanaminium ainsi obtenue est conservée sous une atmosphère d'argon et est utilisée immédiatement dans les synthèses ultérieures.

Le diméthylamino-4 oxo-2 butène-3 oate d'éthyle peut être obtenu de la façon suivante :

A 2100 g d'acétal diéthylique de N,N-diméthylformamide refroidis à une température voisine de 5 ° C, on ajoute en 2 heures, 1500 g de pyruvate d'éthyle en maintenant la température à environ 5 ° C. Le mélange est ensuite agité 3 heures à une température voisine de 20 ° C. L'éthanol réactionnel est évaporé à sec et le résidu obtenu est battu par 12 litres d'éther diéthylique et 30 g de noir 3S puis filtré sur supercel. Le filtrat est repris par 500 cm3 d'eau, la phase organique est décantée et la phase aqueuse est extraite par 3 fois 2000 cm3 de dichlorométhane. Les phases organiques sont réunies, séchées sur sulfate de sodium, filtrées et concentrées à sec à 40 ° C sous pression réduite (20 mm de mercure ; 2,7 kPa). L'huile obtenue est purifiée par distillation ($Eb_{0,1 mmHg}$ = 146-149 ° C). On obtient ainsi 695 g de diméthylamino-4 oxo-2 butène-3 oate d'éthyle utilisé à l'état brut dans les syn thèses ultérieures.

Le phénylthioacétate d'éthyle peut être obtenu de la façon suivante :

A 1 400 cm3 d'une solution éthanolique 2M d'éthylate de sodium, on ajoute, goutte à goutte, en 10 minutes, à une température voisine de 20 °C, 300 g de thiophénol. On introduit ensuite 454 g de bromoacétate d'éthyle en maintenant la température à environ 10 °C. Le précipité obtenu est filtré et la solution éthanolique résiduelle est concentrée à sec à 40 °C sous pression réduite (20 mm de mercure ; 2,7 kPa). On distille et obtient 352 g de phénylthioacétate d'éthyle dont le point d'ébullition est de 125 °C sous 0,75 mm de mercure (0,1 kPa).

EXEMPLE 3

A une solution de 21 g de diméthylamino-2 (oxo-1 tétrahydro-1,2,3,4 naphtylidène-2)-4 butène-2 oate d'éthyle dans 150 cm3 d'éthanol maintenue à reflux, on ajoute, en 2 minutes, 70 cm3 d'une solution aqueuse d'acide chlorhydrique 1N. Le mélange est immédiatement refroidi à une température voisine de 20 à 30 °C et l'éthanol réactionnel est distillé à 40 °C sous pression réduite (20 mm de mercure ; 2,7 kPa). La suspension obtenue est diluée par 50 cm3 d'eau et extraite par 2 fois 50 cm3 d'acétate d'éthyle. Après lavage avec 50 cm3 d'eau et séchage, la phase organique est concentrée à sec à 40 °C sous pression réduite (20 mm de mercure ; 2,7 kPa). Après recristallisation du solide obtenu dans un mélange d'oxyde d'isopropyle et d'acétate d'éthyle (90-10 en volumes) bouillant, on obtient 11,2 g d'hydroxy-2 (oxo-1 tétrahydro-1,2,3,4 naphtylidène-2)-4 butène-2 oate d'éthyle fondant à 117 °C.

Le diméthylamino-2 (oxo-1 tétrahydro-1,2,3,4 naphtylidène-2)-4 butène-2 oate d'éthyle peut être obtenu de la façon suivante :

A un mélange de 100 g de tétrafluoroborate de N-(diméthylamino-3 éthoxycarbonyl-3 propénylidène) N-méthyl méthanaminium dans 500 cm3 d'éthanol et 210 cm3 d'une solution éthanolique 2M d'éthylate de sodium, on ajoute, goutte à goutte, en 45 minutes, à une température voisine de 15 °C, 51 g d'α-tétralone. Le mélange est maintenu pendant 1 heure 45 minutes à une température voisine de 20 °C puis est chauffé pendant 4 heures 45 minutes à 70 °C. L'éthanol réactionnel est évaporé à sec et l'huile obtenue est battue avec 4 fois 300 cm3 d'éther éthylique. Les extraits éthérés sont concentrés à sec sous pression réduite (20 mm de mercure ; 2,7 kPa). Après recristallisation du solide obtenu dans 60 cm3 d'un mélange d'oxyde d'isopropyle et d'acétate d'éthyle (90-10 en volumes) bouillant on obtient 17,5 g de diméthylamino-2 (oxo-1 tétrahydro-1,2,3,4 naphtylidène-2)-4 butène-2 oate d'éthyle fondant à 84 °C.

EXEMPLE 4

A une solution de 13 g de phényl-6 oxo-6 méthylthio-5 diméthylamino-2 hexadiène-2,4 oate d'éthyle dans 100 cm3 d'éthanol, on ajoute, en 10 minutes et en maintenant au reflux, 53 cm3 d'une solution aqueuse 1N d'acide chlorhydrique. Le mélange est immédiatement refroidi et l'éthanol réactionnel est concentré à 40 °C sous pression réduite (20 mm de mercure ; 2,7 kPa). La suspension obtenue est diluée par 100 cm3 d'eau distillée et extraite par 3 fois 100 cm3 d'oxyde de diéthyle. Après lavage à l'eau, les extraits organiques sont concentrés à sec à 40 °C sous pression réduite (20 mm de mercure ; 2,7 kPa). Après recristallisation du solide obtenu dans 45 cm3 d'oxyde d'isopropyle bouillant, on obtient 10,2 g de phényl-6 oxo-6 méthylthio-5 hydroxy-2 hexadiène-2,4 oate d'éthyle fondant à 86 °C.

Le phényl-6 oxo-6 méthylthio-5 diméthylamino-2 hexadiène-2,4 oate d'éthyle peut être obtenu de la façon suivante :

A une solution de 26 g de tétrafluoroborate de N-(diméthylamino-3 éthoxycarbonyl-3 propénylidène) N-méthyl méthanami-nium et de 30 g d'α-méthylthioacétophénone dans 100 cm3 d'éthanol maintenue à une température voisine de 25 °C, on ajoute, goutte à goutte, en 30 minutes, 54 cm3 d'une solution éthanolique 2M d'éthylate de sodium. Le mélange est maintenu pendant 2 heures 10 minutes à une température voisine de 25 °C puis l'éthanol réactionnel est éliminé par distillation à 40 °C sous pression réduite (20 mm de mercure ; 2,7 kPa). Après recristallisation du résidu dans 100 cm3 d'oxyde d'isopropyle bouillant, on obtient 19,2 g de phényl-6 oxo-6 méthylthio-5 diméthylamino-2 hexadiène-2,4 oate d'éthyle fondant à 78 °C.

L'α-méthylthioacétophénone peut être obtenue de la façon suivante :

A une solution de 199 g d'α-bromoacétophénone dans 500 cm3 d'éthanol, on ajoute, en 1 heure 30 minutes, par petites portions et à une température voisine de 20 °C, 70 g de méthanethiolate de sodium. Le mélange est maintenu pendant 2 heures 30 minutes à une température voisine de 20 °C. Le solide en suspension est éliminé par filtration et l'éthanol réactionnel est éliminé par évaporation à sec à 40 °C sous pression réduite (20 mm de mercure ; 2,7 kPa). Le résidu huileux est repris par 400 cm3 d'oxyde de

diéthyle, la phase organique est lavée par 4 fois 200 cm3 d'eau, séchée et concentrée à sec à 40 °C sous pression réduite (20 mm de mercure ; 2,7 kPa). Après distillation, on obtient 98 g d'α-méthylthioacétophénone distillant à 100-102 °C sous une pression de 0,2 mm de mercure (0,027 kPa).

EXEMPLE 5

On opère comme à l'exemple 3, à partir de 4,2 g de diméthylamino-2 (méthoxy-6 oxo-1 tétrahydro-1,2,3,4 naphtylidène-2)-4 butène-2 oate d'éthyle, de 19,1 cm3 d'une solution aqueuse d'acide chlorhydrique 1N et de 19 cm3 d'éthanol. Le mélange est chauffé pendant 5 minutes à ébullition puis refroidi à une température voisine de 20 °C. Après purification par recristallisation dans 120 cm3 d'éthanol bouillant, on obtient 3,4 g d'hydroxy-2 (méthoxy-6 oxo-1 tétrahydro-1,2,3,4 naphtylidène-2)-4 butène-2 oate d'éthyle fondant à 175 °C.

Le diméthylamino-2 (méthoxy-6 oxo-1 tétrahydro-1,2,3,4 naphtylidène-2)-4 butène-2 oate d'éthyle peut être préparé de la manière suivante :

On opère comme à l'exemple 3 pour la préparation du dimé thylamino-2 (oxo-1 tétrahydro-1,2,3,4 naphtylidène-2)-4 butène-2 oate d'éthyle, à partir de 14,3 g de tétrafluoroborate de N-(diméthylamino-3 éthoxycarbonyl-3 propénylidène) N-méthyl méthanaminium, de 30 cm3 d'une solution éthanolique 2M d'éthylate de sodium et de 8,8 g de méthoxy-6 oxo-1 tétrahydro-1,2,3,4 naphtalène dans 72 cm3 d'éthanol. Après purification par chromatographie sur colonne de silice avec un mélange de cyclohexane et d'acétate d'éthyle (50-50 en volumes) comme éluant et précipitation dans 150 cm3 d'oxyde d'éthyle, on obtient 7,2 g de diméthylamino-2 (oxo-1 tétrahydro-1,2,3,4 naphtylidène-2)-4 butène-2 oate d'éthyle fondant à 123 °C.

EXEMPLE 6

On opère comme à l'exemple 3, à partir de 3,8 g de diméthylamino-2 (oxo-2 dihydro-2,3 indénylidène-1)-4 butène-2 oate d'éthyle, de 20 cm3 d'une solution aqueuse d'acide chlorhydrique 1N et de 20 cm3 d'éthanol. Le mélange est chauffé 10 minutes à une température d'environ 60 °C puis est refroidi à environ 20 °C. Après purification par chromatographie sur colonne de silice avec un mélange de cyclohexane et d'acétate d'éthyle (70-30 en volumes) comme éluant et recristallisation dans 52 cm3 d'un mélange de cyclohexane et d'éthanol (90-10 en volumes) bouillant, on obtient 1,5 g d'hydroxy-2 (oxo-2 dihydro-2,3-indénylidène-1)-4 butène-2 oate d'éthyle fondant 137 °C.

Le diméthylamino-2 (oxo-2 dihydro-2,3 indénylidène-1)-4 butène-2 oate d'éthyle peut être préparé de la manière suivante :

On opère comme à l'exemple 3 pour la préparation du diméthylamino-2 (oxo-1 tétrahydro-1,2,3,4 naphtylidène-2)-4 butène-2 oate d'éthyle, à partir de 14,3 g de tétrafluoroborate de N-(diméthylamino-3 éthoxycarbonyl-3 propénylidène) N-méthyl méthanaminium, de 31,2 cm3 d'une solution 1,6M de butyllithium dans l'hexane et de 6,6 g d'indanone-2 dans 100 cm3 de tétrahydrofuranne à une température voisine de 0 °C. Après purification par chromatographie sur colonne de silice avec un mélange de cyclohexane et d'acétate d'éthyle (50-50 en volumes) comme éluant et recristallisation dans 70 cm3 d'oxyde d'isopropyle bouillant, on obtient 4,9 g de diméthylamino-2 (oxo-2 dihydro-2,3 indénylidène-1)-4 butène-2 oate d'éthyle fondant à 93-95 °C.

EXEMPLE 7

On opère comme à l'exemple 3, à partir de 16,8 g de diméthylamino-2 (oxo-1 thia-3 tétrahydro-1,2,3,4 naphtylidène-2)-4 butène-2 oate d'éthyle, de 120 cm3 d'une solution aqueuse d'acide chlorhydrique 1N et de 120 cm3 d'éthanol. Le mélange est agité pendant 15 heures à une température voisine de 20 °C. Après purification par chromatographie sur colonne de silice avec du dichlorométhane comme éluant et recristallisation dans 40 cm3 d'isopropanol bouillant, on obtient 1,5 g d'hydroxy-2 (oxo-1 thia-3 tétrahydro-1,2,3,4 naphtylidène-2)-4 butène-2 oate d'éthyle fondant à 152 °C.

Le diméthylamino-2 (oxo-1 thia-3 tétrahydro-1,2,3,4 naphtylidène-2)-4 butène-2 oate d'éthyle peut être préparé de la manière suivante :

On opère comme à l'exemple 3 pour la préparation du diméthylamino-2 (oxo-1 tétrahydro-1,2,3,4 naphtylidène-2)-4 butène-2 oate d'éthyle, à partir de 15,2 g de tétrafluoroborate de N-(diméthylamino-3 éthoxycarbonyl-3 propénylidène) N-méthyl méthanaminium, de 35 cm3 d'une solution éthanolique 2M

d'éthylate de sodium et de 8,7 g d'oxo-1 thia-3 tétrahydro-1,2,3,4 naphtalène dans 160 cm3 d'éthanol. On obtient 13 g de diméthylamino-2 (oxo-1 thia-3 tétrahydro-1,2,3,4-naphtylidène-2)-4 butène-2 oate d'éthyle utilisé à l'état brut dans les synthèses ultérieures.

L'oxo-1 thia-3 tétrahydro-1,2,3,4 naphtalène peut être préparé selon le procédé suivant :

975 cm3 de nitrobenzène et 292,5 g d'acide polyphosphorique sont chauffés à une température voisine de 70 °C. 97,5 g d'acide benzylthioacétique sont ajoutés au milieu réactionnel. On laisse agiter pendant 26 heures à environ 70 °C puis refroidit à environ 20 °C. 4000 cm3 d'une solution aqueuse saturée de bicarbonate de sodium et 4000 cm3 de dichlorométhane sont ajoutés à la solution. La phase organique est décantée et la phase aqueuse est extraite par 2 fois 200 cm3 de dichlorométhane. Les phases organiques sont réunies, séchées sur sulfate de magnésium anhydre, filtrées et évaporées à 40 °C sous pression réduite (20 mm de mercure ; 2,7 kPa). Le résidu est dissous dans 80 cm3 d'un mélange de cyclohexane et d'acétate d'éthyle (85-15 en volumes) et la solution est versée sur 2 kg de silice contenus dans une colonne de 9 cm de diamètre. On élue avec un mélange de cyclohexane et d'acétate d'éthyle (85-15 en volumes) ; l'éluant correspondant est concentré à sec à 40 °C sous pression réduite (20 mm de mercure ; 2,7 kPa). On obtient ainsi 10 g d'oxo-1 thia-3 tétrahydro-1,2,3,4 naphtalène utilisé à l'état brut dans les synthèses ultérieures.

L'acide benzylthioacétique peut être préparé de la manière suivante :

500 cm3 d'une solution aqueuse de soude 1N refroidie à une température d'environ 5 °C sont ajoutés en 1 heure à 70 g d'acide bromoacétique. Le mélange obtenu est ajouté à une solution de 50 g de phénylméthanethiol, de 500 cm3 d'une solution aqueuse de soude 1N dans 500 cm3 de tétrahydrofuranne puis chauffé à environ 100 °C pendant 90 minutes. Le mélange est refroidi à une température voisine de 5 °C. On ajoute 100 cm3 d'une solution aqueuse d'acide chlorhydrique 12N puis 3000 cm3 de dichlorométhane en 30 minutes. La phase organique est décantée, la phase aqueuse est extraite par 2 fois 200 cm3 de dichlorométhane. Les phases organiques sont réunies, séchées sur du sulfate de magnésium anhydre, filtrées et évaporées à sec à 40 °C sous pression réduite (20 mm de mercure ; 2,7 kPa). Le résidu est recristallisé dans 50 cm3 de cyclohexane bouillant. On obtient 40,5 g d'acide benzylthioacétique fondant à 58-60 °C.

## EXEMPLE 8

On opère comme à l'exemple 4, à partir de 7,8 g de (chloro-4 phényl)-6 diméthylamino-2 méthylthio-5 oxo-6 hexadiène-2,4 oate d'éthyle et de 33 cm3 d'une solution aqueuse d'acide chlorhydrique 1N dans 300 cm3 d'éthanol. Le mélange est chauffé 5 minutes à ébullition puis refroidi à une température voisine de 20 °C. Après purification par recristallisation dans 70 cm3 de cyclohexane bouillant, on obtient 5,6 g de (chloro-4 phényl)-6 hydroxy-2 méthylthio-5 oxo-6 hexadiène-2,4 oate d'éthyle fondant à 88 °C.

Le (chloro-4 phényl)-6 diméthylamino-2 méthylthio-5 oxo-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle, à partir de 11,3 g de tétrafluoroborate de N-(diméthylamino-3 éthoxycarbonyl-3 propénylidène) N-méthyl méthanaminium, de 19,7 cm3 d'une solution éthanolique 2M d'éthylate de sodium et de 7,9 g d'α-méthylthio chloro-4 acétophénone dans 86 cm3 d'éthanol. Après purification par recristallisation dans 130 cm3 de cyclohexane bouillant, on obtient 7,8 g de (chloro-4 phényl)-6 diméthylamino-2 méthylthio-5 oxo-6 hexadiène-2,4 oate d'éthyle fondant à 93 °C.

L'α-méthylthio chloro-4 acétophénone peut être préparée de la manière suivante :

A une solution agitée de 11,7 g d'α-bromo chloro-4 acétophénone dans 100 cm3 de méthanol, on ajoute, en 15 minutes, 3,5 g de méthanethiolate de sodium à une température voisine de 20 °C et on laisse agiter pendant 2 heures à la même température. Le mélange réactionnel est évaporé à sec à 50 °C sous pression réduite (20 mm de mercure ; 2,7 kPa). Le résidu obtenu est dissous dans 50 cm3 de dichlorométhane, la phase organique séchée sur du sulfate de magnésium anhydre, filtrée et concentrée à sec à 40 °C sous pression réduite (20 mm de mercure ; 2,7 kPa). On obtient ainsi 9,9 g d'α-méthylthio chloro-4 acétophénone utilisée à l'état brut dans les synthèses ultérieures.

## EXEMPLE 9

On opère comme à l'exemple 4 à partir de 4,1 g de diméthylamino-2 (méthyl-4 phényl)-6 méthylthio-5 oxo-6 hexadiène-2,4 oate d'éthyle et de 18,5 cm3 d'une solution aqueuse d'acide chlor hydrique 1N dans

18,5 cm3 d'éthanol. Le mélange est chauffé 5 minutes à ébullition puis refroidi à une température voisine de 20°C. Après purification par recristallisation dans 35 cm3 de cyclohexane bouillant, on obtient 2,9 g d'hydroxy-2 (méthyl-4 phényl)-6 méthylthio-5 oxo-6 hexadiène-2,4 oate d'éthyle fondant à 78°C.

Le diméthylamino-2 (méthyl-4 phényl)-6 méthylthio-5-oxo-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle à partir de 23,4 g de tétrafluoroborate de N-(diméthylamino-3 éthoxycarbonyl-3 propénylidène) N-méthyl méthanamium, de 40,8 cm3 d'une solution éthanolique 2M d'éthylate de sodium, et de 14,7 g d'α-méthylthio méthyl-4 acétophénone dans 163 cm3 d'éthanol. Après purification sur colonne de silice avec un mélange de cyclohexane et d'acétate d'éthyle (50-50 en volumes) comme éluant et recristallisation dans 65 cm3 de cyclohexane bouillant, on obtient 5,9 g de diméthylamino-2 (méthyl-4 phényl)-6 méthylthio-5 oxo-6 hexadiène-2,4 oate d'éthyle fondant à 98°C.

L'α-méthylthio méthyl-4 acétophénone peut être préparée de la manière suivante :

On opère comme à l'exemple 4 pour la préparation de l'α-méthylthio acétophénone ; à une solution agitée de 20,8 g d'α-bromo méthyl-4 acétophénone dans 195 cm3 de méthanol, on ajoute, en 30 minutes, 6,8 g de méthanethiolate de sodium, à une température voisine de 20°C et on laisse agiter pendant 2 heures à la même température. On obtient ainsi 16,5 g d'α-méthylthio méthyl-4 acétophénone utilisée à l'état brut dans les synthèses ultérieures.

EXEMPLE 10

On opère comme à l'exemple 4 à partir de 6,1 g de diméthylamino-2 (méthoxy-4 phényl)-6 méthylthio-5 oxo-6 hexadiène-2,4 oate d'éthyle et de 26,2 cm3 d'une solution aqueuse d'acide chlorhydrique 1N dans 26,2 cm3 d'éthanol. Le mélange est chauffé 5 minu tes à ébullition puis refroidi à une température voisine de 20°C. Après purification par recristallisation dans 35 cm3 d'oxyde d'isopropyle bouillant, on obtient 2,7 g d'hydroxy-2 (méthoxy-4 phényl)-6 méthylthio-5 oxo-6 hexadiène-2,4 oate d'éthyle fondant à 88°C.

Le diméthylamino-2 (méthoxy-4 phényl)-6 méthylthio-5 oxo-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle à partir de 13,9 g de tétrafluoroborate de N-(diméthylamino-3 éthoxycarbonyl-3 propénylidène) N-méthyl méthanaminium, de 24,2 cm3 d'une solution éthanolique 2M d'éthylate de sodium et de 9,5 g d'α-méthylthio méthoxy-4 acétophénone dans 97 cm3 d'éthanol. Après purification sur colonne de silice avec un mélange de cyclohexane et d'acétate d'éthyle (50-50 en volumes) comme éluant, on obtient 6,1 g de diméthylamino-2 (méthoxy-4 phényl)-6 méthylthio-5 oxo-6 hexadiène-2,4 oate d'éthyle utilisé à l'état brut dans les synthèses ultérieures.

L'α-méthylthio méthoxy-4 acétophénone peut être préparée de la façon suivante :

On opère comme à l'exemple 4 pour la préparation de l'α-méthylthio acétophénone à partir d'une solution agitée de 11,5 g d'α-bromo méthoxy-4 acétophénone dans 100 cm3 de méthanol, on ajoute en 15 minutes 3,5 g de méthanethiolate de sodium à une température voisine de 20°C et on laisse agiter pendant 12 heures à cette température. On obtient ainsi 9,5 g d'α-méthylthio méthoxy-4 acétophénone utilisé à l'état brut dans les synthèses ultérieures.

EXEMPLE 11

On opère comme à l'exemple 4 à partir de 23,5 g de diméthylamino-2 méthylthio-5 oxo-6 (pipéridino-4 phényl)-6 hexadiène-2,4 oate d'éthyle et de 139 cm3 d'acide chlorhydrique 1N dans 139 cm3 d'éthanol. Le mélange est agité 16 heures à une température voisine de 20°C. Après purification par chromatographie sur colonne de silice avec du dichlorométhane comme éluant, puis par une seconde chromatographie avec un mélange de cyclohexane et d'acétate d'éthyle (50-50 en volumes) comme éluant ; l'éluat correspondant est concentré à sec à 40°C sous pression réduite (20 mm de mercure ; 2,7 kPa). Le résidu est recristallisé dans 100 cm3 d'oxyde d'isopropyle bouillant. On obtient ainsi 3 g d'hydroxy-2 méthylthio-5 oxo-6 (pipéridino-4 phényl)-6 hexadiène-2,4 oate d'éthyle fondant à 65°C.

Le diméthylamino-2 méthylthio-5 oxo-6 (pipéridino-4 phényl)-6 hexadiène-2,4 oate d'éthyle peut être obtenu de la manière suivante :

On opère comme à l'exemple 4 pour la préparation du phényl-6 oxo-6 méthylthio-5 diméthylamino-2 hexadiène-2,4 oate d'éthyle, à partir de 34,4 g de tétrafluoroborate de N-(diméthylamino-3 éthoxycarbonyl-3

propénylidène) N-méthyl méthanaminium, de 60 cm3 d'une solution éthanolique 2M d'éthylate de sodium et de 30 g d'α-méthylthio pipéridino-4 acétophénone dans 300 cm3 d'éthanol. Après purification par chromatographie sur colonne de silice avec un mélange de cyclohexane et d'acétate d'éthyle (50-50 en volumes) comme éluant et recristallisation dans 150 cm3 d'isopropanol bouillant, on obtient 27 g de diméthylamino-2 méthylthio-5 oxo-6 (pipéridino-4 phényl)-6 hexadiène-2,4 oate d'éthyle fondant à 113 °C.

L'α-méthylthio pipéridino-4 acétophénone peut être préparée de la manière suivante :

On opère comme à l'exemple 4 pour la préparation de l'α-méthylthio acétophénone. A une solution agitée de 67 g d'α-chloro pipéridino-4 acétophénone dans 670 cm3 de méthanol, on ajoute, en 90 minutes, 20,1 g de méthanethiolate de sodium à une température voisine de 20 °C et on continue l'agitation 3 heures à la même température. Après purification par recristallisation dans 1400 cm3 d'un mélange d'hexane et de cyclohexane (90-10 en volumes) bouillant, on obtient 55 g d'α-méthylthio pipéridino-4 acétophénone fondant à 66 °C.

L'α-chloro pipéridino-4 acétophénone peut être préparée de la façon suivante :

A une solution agitée de 124 g d'α,α'-dibromo pipéridino-4 acétophénone dans 990 cm3 d'une solution aqueuse d'acide chlorhydrique 12N, on ajoute 620 cm3 de chlorure de titane III en solution aqueuse à 15 %. La solution est chauffée à ébullition pendant 2 heures 30 minutes puis refroidie à une température voisine de 20 °C. Le mélange réactionnel est alcalinisé à pH 11 par 900 cm3 d'une solution aqueuse de soude 10N puis repris par 750 cm3 de dichlorométhane et filtré. La phase organique est décantée, lavée par 2 fois 200 cm3 d'eau, séchée sur sulfate de sodium, filtrée et évaporée à sec à 40 °C sous pression réduite (20 mm de mercure ; 2,7 kPa). Le résidu est recristallisé dans 400 cm3 d'isopropanol bouillant. On obtient 67 g d'α-chloro pipéridino-4 acétophénone fondant à 125 °C.

L'α,α'-dibromo pipéridino-4 acétophénone peut être préparée de la façon suivante :

A une solution agitée de 99,5 g de pipéridino-4 acétophénone dans 200 cm3 d'une solution aqueuse d'acide bromhy-drique 8,6N, on ajoute, en 40 minutes, à une température voisine de 20 °C, un mélange de 50 cm3 de brome et de 75 cm3 d'acide bromhydrique en conservant la température à environ 20 °C. L'agitation est maintenue 2 heures à la même température. Le mélange réactionnel est filtré, le gâteau lavé par 300 cm3 d'eau. Le solide est broyé et repris par 800 cm3 de dichlorométhane. La solution obtenue est alcalinisée jusqu'à pH 11 par 200 cm3 de soude 10N et reprise par 600 cm3 d'eau. La phase organique est décantée, séchée sur sulfate de magnésium anhydre, filtrée et évaporée à sec à 40 °C sous pression réduite (20 mm de mercure ; 2,7 kPa). Le résidu est recristallisé dans 2160 cm3 d'éthanol bouillant. On obtient 124 g d'α,α'-dibromo pipéridino-4 acétophénone fondant à 134 °C.

EXEMPLE 12

On opère comme à l'exemple 4, à partir de 4,9 g de diméthylamino-2 (isopropylènedioxy-3,4 phényl)-6 méthylthio-5 oxo-6 hexadiène-2,4 oate d'éthyle et de 20 cm3 d'une solution aqueuse d'acide chlorhydrique 1N dans 20 cm3 d'éthanol. Le mélange est chauffé 5 minutes à ébullition puis refroidi à une température voisine de 20 °C. Après purification par recristallisation dans 70 cm3 d'oxyde d'isopropyle bouillant, on obtient 1,1 g d'hydroxy-2 (isopropylènedioxy-3,4 phényl)-6 méthylthio-5 oxo-6 hexadiène-2,4 oate d'éthyle fondant à 134 °C.

Le diméthylamino-2 (isopropylènedioxy-3,4 phényl)-6 méthylthio-5 oxo-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle, à partir de 7,2 g de tétrafluoroborate de N-(diméthylamino-3 éthoxycarbonyl-3 propénylène) N-méthyl méthanaminium, de 12,5 cm3 d'une solution éthanolique 2M d'éthylate de sodium et de 6 g de diméthyl-2,2 (méthylthio-2 acétyl)-5 benzodioxole-1,3 dans 50 cm3 d'éthanol. Après purification sur colonne de silice avec un mélange de cyclohexane et d'acétate d'éthyle (50-50 en volumes) comme éluant, on obtient 3,9 g de diméthylamino-2 (isopropylènedioxy-3,4 phényl)-6 méthylthio-5 oxo-6 hexadiène-2,4 oate d'éthyle utilisé à l'état brut dans les synthèses ultérieures.

Le diméthyl-2,2 (méthylthio-2 acétyl)-5 benzodioxole-1,3 peut être préparé de la manière suivante :

On opère comme à l'exemple 4 pour la préparation de l'α-méthylthioacétophénone, à partir d'une solution agitée de 14,9 g de (bromo-2 acétyl)-5 diméthyl-2,2 benzodioxole-1,3 dans 150 cm3 de méthanol. On ajoute, en 5 minutes, 3,8 g de méthanethiolate de sodium, à une température voisine de 20 °C et on laisse agiter 15 heures à la même température. On obtient ainsi 11 g de diméthyl-2,2 (méthylthio-2 acétyl)-5 benzodioxole-1,3 utilisé à l'état brut dans les synthèses ultérieures.

Le (bromo-2 acétyl)-5 diméthyl-2,2 benzodioxole-1,3 peut être préparé de la manière suivante :

A une solution agitée de 5,8 g d'acétyl-5 diméthyl-2,2 benzodioxole-1,3 dans 100 cm3 de tétrachlorure

de carbone, on ajoute en 5 minutes, 1,5 cm3 de brome à une température voisine de 20 °C et on laisse agiter 15 heures à la même température. Le mélange réactionnel est repris pas 150 cm3 d'eau. La phase organique est décantée, séchée sur sulfate de magnésium anhydre, filtrée et évaporée à sec à 40 °C sous pression réduite (20 mm de mercure ; 2.7 kPa). On obtient 7,1 g de (bromo-2 acétyl)-5 diméthyl-2,2 benzodioxole-1,3 utilisé à l'état brut dans les synthèses ultérieures.

L'acétyl-5 diméthyl-2,2 benzodioxole-1,3 peut être préparé selon la méthode décrite par G. BENOIT et B. MILLET, Bull. Soc. Chim. Fr. 1960, 638.

## EXEMPLE 13

On opère comme à l'exemple 4, à partir de 1,9 g de (dihydroxy-3,4 phényl)-6 diméthylamino-2 méthylthio-5 oxo-6 hexadiène-2,4 oate d'éthyle et de 10,8 cm3 d'une solution aqueuse d'acide chlorhydrique 1N dans 10,8 cm3 d'éthanol. Le mélange est chauffé 5 minutes à ébullition puis refroidi à une température voisine de 20 °C. Après purification par cristallisation dans 100 cm3 d'oxyde d'isopropyle, on obtient 0,6 g de (dihydroxy-3,4 phényl)-6 hydroxy-2 méthylthio-5 oxo-6 hexadiène-2,4 oate d'éthyle fondant à 156 °C.

Le (dihydroxy-3,4 phényl)-6 diméthylamino-2 méthylthio-5 oxo-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle, à partir de 11,4 g de tétrafluoroborate de N-(diméthylamino-3 éthoxycarbonyl-3 propénylidène) N-méthyl méthanaminium, de 60 cm3 d'une solution éthanolique 2M d'éthylate de sodium et de 7,9 g de (méthylthio-2 acétyl)-4 pyrocatéchol dans 60 cm3 d'éthanol. Après purification sur colonne de silice avec un mélange de cyclohexane et d'acétate d'éthyle (50-50 en volumes) comme éluant, on obtient 2,4 g de (dihydroxy-3,4 phényl)-6 diméthylamino-2 méthylthio-5 oxo-6 hexadiène-2,4 oate d'éthyle fondant à 144 °C.

Le (méthylthio-2 acétyl)-4 pyrocatéchol peut être préparé de la manière suivante :

On opère comme à l'exemple 4 pour la préparation de l'α-méthylthioacétophénone, à partir d'une solution agitée de 18,6 g de (chloro-2 acétyl)-4 pyrocatéchol dans 200 cm3 de méthanol. On ajoute, en 15 minutes, 7 g de méthanethiolate de sodium, à une température voisine de 20 °C et on laisse agiter 3 heures à la même température. On obtient ainsi 14,5 g de (méthylthio-2 acétyl)-4 pyrocatéchol fondant à 107 °C.

## EXEMPLE 14

On opère comme à l'exemple 4, à partir de 8 g de diméthylamino-2 méthoxy-5 oxo-6 phényl-6 hexadiène-2,4 oate d'éthyle et de 40 cm3 d'une solution aqueuse d'acide chlorhydrique 1N dans 40 cm3 d'éthanol. Le mélange est chauffé 5 minutes à ébullition puis refroidi à une température voisine de 20 °C. Après purification par chromatographie sur colonne de silice avec un mélange de cyclohexane et d'acétate d'éthyle (50-50 en volumes) comme éluant et recristallisation dans 70 cm3 d'oxyde d'isopropyle bouillant, on obtient 4 g d'hydroxy-2 méthoxy-5 oxo-6 phényl-6 hexadiène-2,4 oate d'éthyle fondant à 84 °C.

Le diméthylamino-2 méthoxy-5 oxo-6 phényl-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle, à partir de 11,4 g de tétrafluoroborate de N-(diméthylamino-3 éthoxycarbonyl-3 propénylidène) N-méthyl méthanaminium, de 20 cm3 d'une solution éthanolique 2M d'éthylate de sodium et de 6 g d'α-méthoxyacétophénone dans 50 cm3 d'éthanol. Après purification par chromatographie sur colonne de silice avec un mélange de dichlorométhane et d'acétate d'éthyle (50-50 en volumes) comme éluant, on obtient 8,1 g de diméthylamino-2 méthoxy-5 oxo-6 phényl-6 hexadiène-2,4 oate d'éthyle utilisé à l'état brut dans les synthèses ultérieures.

L'α-méthoxyacétophénone est préparée selon R.B. MOFFETT et R.L. SHRINER, Org. Synth. 3 , 562 (1955).

## EXEMPLE 15

On opère comme à l'exemple 4, à partir de 6,9 g de diméthylamino-2 méthylthio-5 (naphtyl-2)-6 oxo-6

hexadiène-2,4 oate d'éthyle, de 29 cm3 d'une solution aqueuse d'acide chlorhydrique 12N et de 29 cm3 d'éthanol. Le mélange est chauffé 5 minute à ébullition puis est refroidi à une température d'environ 20° C. Après purification par recristallisation dans 300 cm3 d'oxyde de diisopropyle bouillant, on obtient 2,7 g d'hydroxy-2 méthylthio-5 (naphtyl-2)-6 oxo-6 hexadiène-2,4 oate d'éthyle fondant à 117° C.

Le diméthylamino-2 méthylthio-5 (naphtyl-2)-6 oxo-6 hexadiène-2,4 oate d'éthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 10,6 g de tétrafluoroborate de N-(diméthylamino-3 éthoxycarbonyl-3 propénylidène) N-méthyl méthanaminium, de 18,5 cm3 d'une solution éthanolique 2M d'éthylate de sodium et de 8 g de (méthylthio-2 acétyl)-2 naphtalène dans 50 cm3 d'éthanol. Après purification par chromatographie sur colonne de silice avec un mélange de cyclohexane et d'acétate d' éthyle (50-50 en volumes) comme éluant, on obtient 5 g de diméthylamino-2 méthylthio-5 (naphtyl-2)-6 oxo-6 hexadiène-2,4 oate d'éthyle, sous forme d'une huile orange, utilisé à l'état brut dans les synthèses ultérieures.

Le (méthylthio-2 acétyl)-2 naphtalène peut être préparé de la façon suivante :

On opère comme à l'exemple 4 pour la préparation de l'α-méthylthio acétophénone, à partir de 12,5 g de (bromo-2 acétyl)-2 naphtalène et de 3,5 g de méthanethiolate de sodium dans 100 cm3 d'éthanol. On obtient ainsi 8 g de (méthylthio-2 acétyl)-2 naphtalène utilisé à l'état brut dans les synthèses ultérieures.

EXEMPLE 16

On opère comme à l'exemple 4, à partir de 9 g de diméthylamino-2 (fluoro-4 phényl)-6 méthylthio-5 oxo-6 hexadiène-2,4 oate d'éthyle, de 36 cm3 d'une solution aqueuse d'acide chlorhydrique 12N et de 100 cm3 d'éthanol. Le mélange est chauffé 5 minutes à ébullition puis est refroidi à une température d'environ 20° C. Après purification par recristallisation dans 50 cm3 de cyclohexane bouillant, on obtient 3,2 g de (fluoro-4 phényl)-6 hydroxy-2 méthylthio-5 oxo-6 hexadiène-2,4 oate d'éthyle fondant à 95° C.

Le diméthylamino-2 (fluoro-4 phényl)-6 méthylthio-5 oxo-6 hexadiène-2,4 oate d'éthyle peut être préparé de la manière suivante :

On opère comme à l'exemple 4, pour la préparation du phényl-6 oxo-6 méthylthio-5 diméthylamino-2 hexadiène-2,4 oate d'éthyle à partir de 10 g de tétrafluoroborate de N-(diméthylamino-3 éthoxycarboyl-3 propénylidène) N-méthyl méthanaminium, de 21 cm3 d'une solution éthanolique 2M d'éthylate de sodium et de 6,5 g d'α-méthylthio fluoro-4 acétophénone dans 100 cm3 d'éthanol. Après purification par chromatographie sur colonne de silice avec un mélange de cyclohexane et d'acétate d'éthyle (50-50 en volumes) comme éluant, on obtient 9 g de diméthylamino-2 (fluoro-4 phényl)-6 méthylthio-5 oxo-6 hexadiène-2,4 oate d'éthyle, sous forme d'une huile orange, utilisé à l'état brut dans les synthèses ultérieures.

L'α-méthylthio fluoro-4 acétophénone peut être préparée de la façon suivante :

On opère comme à l'exemple 4 pour la préparation de l'α-méthylthio-2 acétophénone, à partir de 25 g d'α-bromo p-fluoro acétophénone et de 10,3 g de méthanethiolate de sodium dans 130 cm3 d'éthanol. Après purification par chromatographie sur colonne de silice avec un mélange de cyclohexane et d'acétate d'éthyle (50-50 en volumes) comme éluant, on obtient 21,9 g d'α-méthylthio fluoro-4 acétophénone utilisée à l'état brut dans les synthèses ultérieures.

EXEMPLE 17

On opère comme à l'exemple 4, à partir de 7,7 g de diméthylamino-2 (bromo-4 phényl)-6 méthylthio-5 oxo-6 hexadiène-2,4 oate d'éthyle, de 25 cm3 d'une solution aqueuse d'acide chlorhydrique 12N et de 80 cm3 d'éthanol. Le mélange est chauffé 5 minutes à ébullition puis est refroidi à une température d'environ 20° C. Après purification par chromatographie sur colonne de silice avec un mélange de cyclohexane et d'acétate d'éthyle (80-20 puis 50-50 en volumes) comme éluant et recristallisation dans 40 cm3 de cyclohexane bouillant, on obtient 2,5 g de (bromo-4 phényl)-6 hydroxy-2 méthylthio-5 oxo-6 hexadiène-2,4 oate d'éthyle fondant à 84° C.

Le diméthylamino-2 (bromo-4 phényl)-6 méthylthio-5 oxo-6 hexadiène-2,4 oate d'éthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 8,6 g d'α-méthylthio bromo-4 acétophénone dans 100 cm3 d'éthanol. Après purification par

recristallisation dans 100 cm3 de cyclohexane, on obtient 7,7 g de diméthylamino-2 (bromo-4 phényl)-6 méthylthio-5 oxo-6 hexadiène-2,4 oate d'éthyle fondant à 107° C.

L'α-méthylthio bromo-4 acétophénone peut être préparée de la façon suivante :

On opère comme à l'exemple 4 pour la préparation de l'α-méthylthio acétophénone, à partir de 30 g d'α-p-dibromo acétophénone et de 7,6 g de méthanethiolate de sodium dans 100 cm3 d'éthanol. Après purification par chromatographie sur colonne de silice avec un mélange de cyclohexane et de dichlorométhane (50-50 en volumes) comme éluant, on obtient 22,4 g d'α-méthylthio bromo-4 acétophénone utilisée à l'état brut dans les synthèses ultérieures.

EXEMPLE 18

On opère comme à l'exemple 4, à partir de 2,7 g de diméthylamino-2 (biphényl-4)-6 méthylthio-5 oxo-6 hexadiène-2,4 oate d'éthyle, de 10,7 cm3 d'une solution aqueuse d'acide chlorhydrique 12N et de 10,7 cm3 d'éthanol. Le mélange est chauffé 5 minutes à ébullition puis est refroidi à une température d'environ 20° C. Après purification par recristallisation dans 100 cm3 d'oxyde de diisopropyle bouillant, on obtient 1,1 g d'hydroxy-2 (biphénylyle-4)-6 méthylthio-5 oxo-6 hexadiène-2,4 oate d'éthyle fondant à 125° C.

Le diméthylamino-2 (biphényl-4)-6 méthylthio-5 oxo-6 hexadiène-2,4 oate d'éthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 6 g de tétrafluoroborate de N-(diméthylamino-3 éthoxycarbonyl-3 propénylidène) N-méthyl méthanaminium, de 10,5 cm3 d'une solution éthanolique 2M d'éthylate de sodiume et de 5,1 g d'α-méthylthio phényl-4 acétophénone dans 150 cm3 d'éthanol. Après purification par chromatographie sur colonne de silice avec un mélange de cyclohexane et d'acétate d'éthyle (50-50 en volumes) comme éluant, on obtient 2,8 g de diméthylamino-2 (biphénylyl-4)-6 méthylthio-5 oxo-6 hexadiène-2,4 oate d'éthyle, sous forme d'une huile orange, utilisé à l'état brut dans les synthèses ultérieures.

L'α-méthylthio phényl-4 acétophénone peut être préparée de la façon suivante :

On opère comme à l'exemple 4 pour la préparation de l'α-méthylthio acétophénone, à partir de 9,6 g d'α-bromo phényl-4 acétophénone et de 2,4 g de méthanethiolate de sodium dans 100 cm3 d'éthanol. On obtient 5,1 g d'α-méthylthio phényl-4 acétophénone, fondant à 92° C, utilisée à l'état brut dans les synthèses ultérieures.

EXEMPLE 19

On opère comme à l'exemple 4, à partir de 5,5 g de diméthylamino-2 (diméthylamino-4 phényl)-6 méthylthio-5 oxo-6 hexadiène-2,4 oate d'éthyle, de 38 cm3 d'une solution aqueuse d'acide chlorhydrique 12N et de 50 cm3 d'éthanol. Le mélange est chauffé 5 minutes à ébullition puis est refroidi à une température d'environ 20° C. Après purification par chromatographie sur colonne de silice avec un mélange de cyclohexane et d'acétate d'éthyle (60-40 en volumes), on obtient 3,9 g de (diméthylamino-4 phényl)-6 hydroxy-2 méthylthio-5 oxo-6 hexadiène-2,4 oate d'éthyle sous forme d'huile brune (Rf = 0,2 ; support : gel de silice ; éluant : cyclo hexane/acétate d'éthyle 60-40 en volumes).

Le diméthylamino-2 (diméthylamino-4 phényl)-6 méthylthio-5 oxo-6 hexadiène-2,4 oate d'éthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 8,6 g de tétrafluoroborate de N-(diméthylamino-3 éthoxycarbonyl-3 propénylidène) N-méthyl méthanaminium, de 15 cm3 d'une solution éthanolique 2M d'éthylate de sodium et de 5,8 g d'α-méthylthio diméthylamino-4 acétophénone dans 60 cm3 d'éthanol. Après purification par chromatographie sur colonne de silice avec un mélange de cyclohexane et d'acétate d' éthyle (50-50 en volumes) comme éluant, on obtient 5,5 g de diméthylamino-2 (diméthylamino-4 phényl)-6 méthylthio-5 oxo-6 hexadiène-2,4 oate d'éthyle, sous forme d'une huile orange, utilisé à l'état brut dans les synthèses ultérieures.

L'α-méthylthio diméthylamino-4 acétophénone peut être préparée de la façon suivante :

On opère comme à l'exemple 4 pour la préparation de l'α-méthylthio acétophénone, à partir de 12,5 g d'α-bromo diméthylamino-4 acétophénone et de 3,6 g de méthanethiolate de sodium dans 65 cm3 d'éthanol. On obtient 9,7 g d'α-méthylthio diméthylamino-4 acétophénone, fondant à 65° C, utilisée à l'état brut dans les synthèses ultérieures.

EXEMPLE 20

On opère comme à l'exemple 4, à partir de 7,3 g de diméthylamino-2 (chloro-3 phényl)-6 méthylthio-5 oxo-6 hexadiène-2,4 oate d'éthyle, de 30,9 cm3 d'une solution aqueuse d'acide chlorhydrique 12N et de 62 cm3 d'éthanol. Le mélange est chauffé 5 minutes à ébullition puis est refroidi à une température d'environ 20° C. Après purification par recristallisation dans 71 cm3 de cyclohexane bouillant, on obtient 6,6 g de (chloro-3 phényl)-6 hydroxy-2 méthylthio-5 oxo-6 hexadiène-2,4 oate d'éthyle fondant à 90° C.

Le diméthylamino-2 (chloro-3 phényl)-6 méthylthio-5 oxo-6 hexadiène-2,4 dioate de diéthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 11,5 g de tétrafluoroborate de N-(diméthylamino-3 éthoxycarbonyl-3 propénylidène) N-méthyl méthanaminium, de 20 cm3 d'une solution éthanolique 2M d'éthylate de sodium et de 8 g d'α-méthylthio chloro-3 acétophénone dans 80 cm3 d'éthanol. Après purification par chromatographie sur colonne de silice avec un mélange de cyclohexane et d'acétate d'éthyle (50-50 en volumes) comme éluant, on obtient 7,3 g de diméthylamino-2 (chloro-3 phényl)-6 méthylthio-5 oxo-6 hexadiène=2,4 oate d'éthyle, sous forme d'une huile orange, utilisé à l'état brut dans les synthèses ultérieures.

L'α-méthylthio chloro-3 acétophénone peut être préparée de la façon suivante :

On opère comme à l'exemple 4 pour la préparation de l'α-méthylthio acétophénone, à partir de 22,3 g d'α-bromo chloro-3 acétophénone et de 6,8 g de méthanethiolate de sodium dans 223 cm3 d'éthanol. On obtient ainsi 17,3 g d'α-méthylthio chloro-3 acétophénone utilisée à l'état brut dans les synthèses ultérieures.

Les médicaments selon l'invention sont constitués par un composé de formule (I) à l'état pur ou sous forme d'une composition dans laquelle il est associé à tout autre produit pharmaceutiquement compatible, pouvant être inerte ou physiologiquement actif. Ces médicaments selon l'invention peuvent être utilisés par voie orale, parentérale, rectale ou topique.

Comme compositions solides pour administration orale, peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles comme antiinflammatoires, comme agents protecteurs notamment sur le tractus gastroinstestinal, et pour le traitement de l'asthme, des maladies allergiques, du psoriasis, de l'arthrite rhumatoïde et des fibroses notamment des fibroses hépathiques.

Les doses dépendent de l'effet recherché, de la durée du traitement et de la voie d'administration utilisée ; elles sont généralement comprises entre 0,1 g et 5 g par jour par voie orale pour un adulte avec des doses unitaires allant de 20 à 200 mg de substance active.

D'une façon générale, le médecin déterminera la posologie appropriée en fonction de l'âge, du poids et

de tous les autres facteurs propres au sujet à traiter.

Les exemples suivants illustrent des compositions selon l'invention :

## EXEMPLE A

On prépare, selon la technique habituelle, des gélules dosées à 50 mg de produit actif ayant la composition suivante :

| | |
|---|---|
| - hydroxy-2 méthylthio-5 oxo-6 (pipéridino-4 phényl)-6 hexadiène-2,4 oate d'éthyle ..... | 50 mg |
| - cellulose ..... | 18 mg |
| - lactose ..... | 55 mg |
| - silice colloïdale ..... | 1 mg |
| - carboxyméthylamidon sodique ..... | 10 mg |
| - talc ..... | 10 mg |
| - stéarate de magnésium ..... | 1 mg |

## EXEMPLE B

On prépare, selon la technique habituelle, des comprimés dosés à 50 mg de produit actif ayant la composition suivante :

| | |
|---|---|
| - hydroxy-5 phénylthio-2 pentadiène-2,4 oate d'éthyle ..... | 50 mg |
| - lactose ..... | 104 mg |
| - cellulose ..... | 40 mg |
| - polyvidone ..... | 10 mg |
| - carboxyméthylamidon sodique ..... | 22 mg |
| - talc ..... | 10 mg |
| - stéarate de magnésium ..... | 2 mg |
| - silice colloïdale ..... | 2 mg |
| - mélange d'hydroxyméthylcellulose, glycérine, oxyde de titane (72-3,5-24,5) ..... q. s. p. | 1 comprimé pelliculé terminé à 245 mg |

## EXEMPLE C

On prépare une solution injectable contenant 10 mg de produit actif ayant la composition suivante :

| | |
|---|---|
| - acide hydroxy-2 phénylthio-5 éthoxycarbonyl-5 pentadiène-2,4 oïque ..... | 10 mg |
| - acide benzoïque ..... | 80 mg |
| - alcool benzylique ..... | 0,06 cm3 |
| - benzoate de sodium ..... | 80 mg |
| - éthanol à 95 % ..... | 0,4 cm3 |
| - hydroxyde de sodium ..... | 24 mg |
| - propylène glycol ..... | 1,6 cm3 |
| - eau ..... q. s. p. | 4 cm3 |

**Revendications**

1 - Les composés de formule :

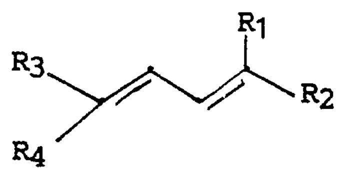

(I)

dans laquelle, $R_1$ représente un radical hydroxy ou acétoxy, $R_2$ représente un atome d'hydrogène, un radical carboxy, alcoxycarbonyle, phényle ou benzoyle et
- soit $R_3$ représente un radical alkylthio ou alcoxy et $R_4$ représente un radical naphtoyle, benzoyle ou benzoyle substitué par un ou plusieurs atomes d'halogène, un ou plusieurs radicaux alkyle, alcoxy, phényle, phénoxy, pipéridino, diméthylamino ou hydroxy ou en positions -3 et -4 par un radical isopropylènedioxy,
- soit $R_3$ et $R_4$ forment ensemble avec l'atome de carbone auquel ils sont rattachés un des cycles de formules :

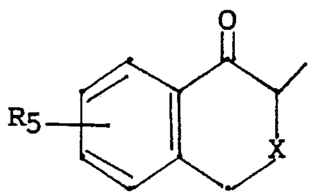

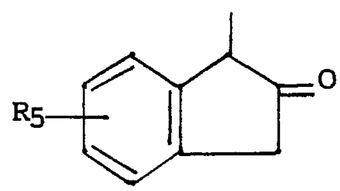

dans lesquelles $R_5$ représente un atome d'hydrogène ou un radical alcoxy et X représente un radical méthylène ou un atome de soufre, étant entendu que lorsque $R_1$ représente un radical acétoxy, $R_4$ ne peut pas représenter un radical benzoyle substitué par un ou plusieurs radicaux hydroxy, que, sauf mention contraire, dans les définitions qui précèdent les radicaux alkyle et alcoxy, et les portions alkyle et alcoxy contiennent 1 à 4 atomes de carbone en chaîne droite ou ramifiée ainsi que la forme tautomère de ces composés lorsque $R_1$ représente un radical hydroxy.

2 - Procédé de préparation d'un composé selon la revendication 1 dans la formule duquel $R_1$ représente le radical hydroxy, $R_2$ représente un atome d'hydrogène ou un radical alcoxycarbonyle, phényle ou benzoyle et $R_3$ et $R_4$ sont définis comme dans la revendication 1 caractérisé en ce que l'on hydrolyse en milieu acide une énamine de formule :

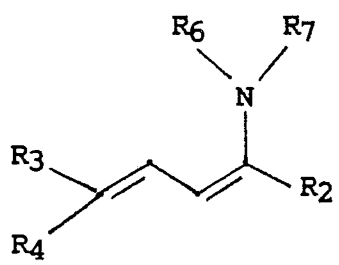

(II)

dans laquelle $R_2$, $R_3$ et $R_4$ ont les significations mentionnées ci-dessus et $R_6$ et $R_7$ représentent des radicaux alkyle ou $R_6$ et $R_7$ forment ensemble avec l'atome d'azote auquel ils sont rattachés un hétérocycle puis isole le produit obtenu.

3 - Les énamines de formule :

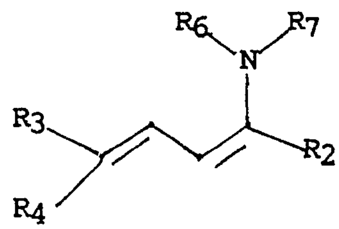

(II)

dans laquelle $R_2$ représente un atome d'hydrogène ou un radical alcoxycarbonyle, phényle ou benzoyle et
- soit $R_3$ représente un radical alkylthio ou alcoxy et $R_4$ représente un radical naphtoyle, benzoyle ou benzoyle substitué par un ou plusieurs atomes d'halogène, un ou plusieurs radicaux alkyle, alcoxy, phényle, phénoxy, pipéridino, diméthylamino ou hydroxy ou en positions -3 et -4 par un radical isopropylènedioxy,
- soit $R_3$ et $R_4$ forment ensemble avec l'atome de carbone auquel ils sont rattachés un des cycles de formules :

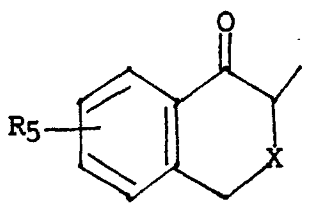

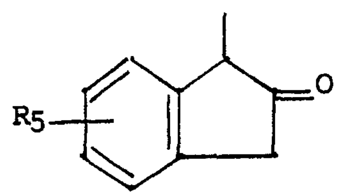

dans lesquelles $R_5$ représente un atome d'hydrogène ou un radical alcoxy et X représente un radical méthylène ou un atome de soufre, $R_6$ et $R_7$ représentent des radicaux alkyle ou $R_6$ et $R_7$ forment ensemble avec l'atome d'azote auquel ils sont rattachés un hétérocycle, étant entendu que, sauf mention contraire, dans les définitions qui précèdent, les radicaux alkyle et alcoxy et les portions alkyle et alcoxy contiennent 1 à 4 atomes de carbone en chaîne droite ou ramifiée.

4 - Procédé de préparation d'un composé selon la revendication 1 dans la formule duquel $R_1$ représente un radical hydroxy, $R_2$ représente un radical alcoxycarbonyle, $R_3$ et $R_4$ sont définis comme dans la revendication 1 caractérisé en ce que l'on hydrolyse en milieu acide une énamine de formule :

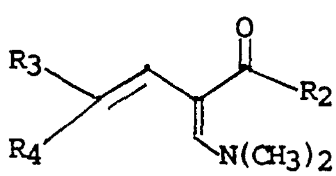

(VI)

dans laquelle $R_2$, $R_3$ et $R_4$ ont les significations mentionnées ci-dessus puis isole le produit.

5 - Les énamines de formule :

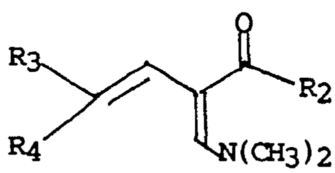

(VI)

dans laquelle $R_2$ représente un radical alcoxycarbonyle et
- soit $R_3$ représente un radical alkylthio ou alcoxy et $R_4$ représente un radical naphtoyle, benzoyle ou benzoyle substitué par un ou plusieurs atomes d'halogène, un ou plusieurs radicaux alkyle, alcoxy, phényle, phénoxy, pipéridino, diméthylamino ou hydroxy ou en positions -3 et -4 par un radical isopropylènedioxy,
- soit $R_3$ et $R_4$ forment ensemble avec l'atome de carbone auquel ils sont rattachés un des cycles de formules :

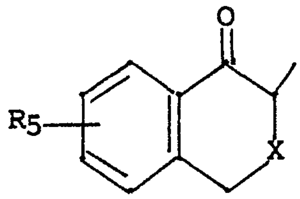 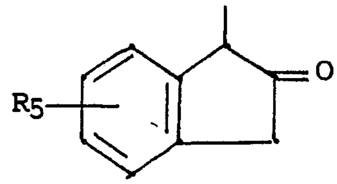

dans lesquelles $R_5$ représente un atome d'hydrogène ou un radical alcoxy et X représente un radical méthylène ou un atome de soufre,

6 - Procédé de préparation d'un composé selon la revendication 1 dans la formule duquel $R_1$ représente le radical hydroxy, $R_2$ représente le radical carboxy, $R_3$ et $R_4$ sont définis comme dans la revendication 1 caractérisé en ce que l'on saponifie l'ester énol de formule :

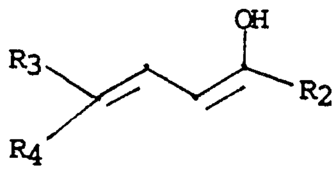 (VII)

dans laquelle $R_2$ représente un radical alcoxycarbonyle et $R_3$, $R_4$ sont définis comme dans la revendication 1 et isole le produit.

7 - Procédé de préparation d'un composé selon la revendication 1 dans la formule duquel $R_1$ représente le radical acétoxy et $R_2$, $R_3$ et $R_4$ sont définis comme dans la revendication 1 étant entendu que $R_3$ ne peut pas représenter un radical benzoyle substitué par un ou plusieurs radicaux hydroxy caractérisé en ce que l'on fait réagir le chlorure d'acétyle sur le composé de formule (I) correspondant dans laquelle $R_1$ représente le radical hydroxy et isole le produit.

8 - Médicaments contenant au moins une substance active et éventuellement un ou plusieurs diluants ou adjuvants compatibles et pharmaceutiquement acceptables caractérisé en ce que la substance active est un composé selon la revendication 1.

Revendications pour les Etats contractants suivants: GR, ES

1 - Procédé de préparation des composés de formule :

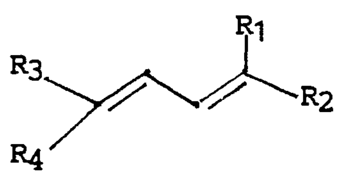 (I)

dans laquelle, $R_1$ représente un radical hydroxy ou acétoxy, $R_2$ représente un atome d'hydrogène, un radical carboxy, alcoxycarbonyle, phényle ou benzoyle et

- soit $R_3$ représente un radical alkylthio ou alcoxy et $R_4$ représente un radical naphtoyle, benzoyle ou benzoyle substitué par un ou plusieurs atomes d'halogène, un ou plusieurs radicaux alkyle, alcoxy, phényle, phénoxy, pipéridino, diméthylamino ou hydroxy ou en positions -3 et -4 par un radical isopropylènedioxy,
- soit $R_3$ et $R_4$ forment ensemble avec l'atome de carbone auquel ils sont rattachés und es cycles de formules :

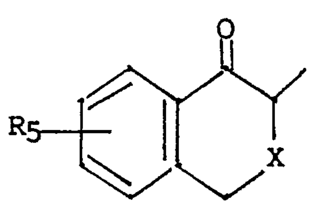 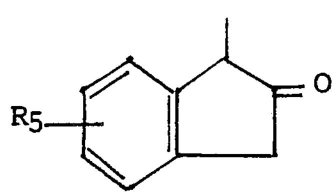

dans lesquelles $R_5$ représente un atome d'hydrogène ou un radical alcoxy et X représente unradical méthylène ou un atome de soufre, étant entendu que lorsque $R_1$ représente un radical acétoxy, $R_4$ ne peut pas représenter un radical benzoyle substitué par un ou plusieurs radicaux hydroxy, que, sauf mention contraire, dans les définitions qui précèdent et qui suivent les radicaux alkyle et alcoxy, et les portions alkyle et alcoxy contiennent 1 à 4 atomes de carbone en chaîne droite ou ramifiée ainsi que la forme tautomère de ces composés lorsque $R_1$ représente un radical hydroxy, caractérisé en ce que :

A - Pour la préparation d'un composé de formule (I) dans laquelle $R_1$ représente le radical hydroxy, $R_2$ représente un atome d'hydrogène ou un radical alcoxycarbonyle, phényle ou benzoyle et $R_3$ et $R_4$ sont définis comme précédemment, on hydrolyse en milieu acide une énamine de formule :

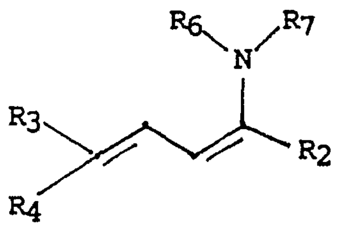

(II)

dans laquelle $R_2$, $R_3$ et $R_4$ sont définis comme précédemment et $R_6$ et $R_7$ représentent des radicaux alkyle ou $R_6$ et $R_7$ forment ensemble avec l'atome d'azote auquel ils sont rattachés un hétérocycle puis isole le produit obtenu.

B - Pour la préparation d'un composé de formule (I) dans laquelle $R_1$ représente le radical hydroxy, $R_2$ représente un radical alcoxycarbonyle, $R_3$ et $R_4$ sont définis comme précédemment, on hydrolyse en milieu acide une énamine de formule :

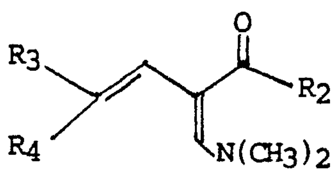

(VI)

dans laquelle $R_2$, $R_3$ et $R_4$ sont définis comme précédemment puis isole le produit obtenu.

C - Pour la préparation d'un composé de formule (I) dans laquelle $R_1$ représente le radical hydroxy, $R_2$ représente le radical carboxy, $R_3$ et $R_4$ sont définis comme précédemment, on saponifie l'ester énol de formule :

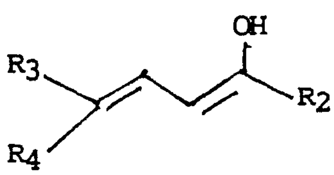

(VII)

dans laquelle $R_2$ représente un radical alcoxycarbonyle et $R_3$, $R_4$ sont définis comme précédemment et isole le produit obtenu.

D - Pour la préparation d'un composé de formule (I) dans laquelle $R_1$ représente le radical acétoxy et $R_2$, $R_3$ et $R_4$ sont définis comme précédemment, étant entendu que $R_3$ ne peut pas représenter un radical benzoyle substitué par un ou plusieurs radicaux hydroxy, on fait réagir le chlorure d'acétyle sur le composé de formule (I) correspondant pour lequel $R_1$ représente le radical hydroxy et isole le produit obtenu.

2 - Procédé de préparation des énamines de formule :

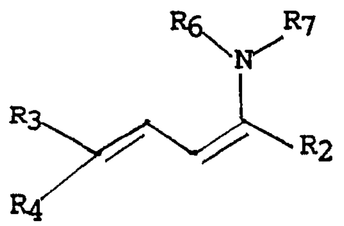

(II)

dans laquelle $R_2$ représente un atome d'hydrogène ou un radical alcoxycarbonyle, phényle ou benzoyle, $R_3$ et $R_4$ ont les mêmes significations que dans la formule (I) de la revendication 1, $R_6$ et $R_7$ représentent des radicaux alkyle ou $R_6$ et $R_7$ forment ensemble avec l'atome d'azote auquel ils sont rattachés un hétérocycle caractérisé en ce que l'on fait réagir un sel de vinamidinium de formule :

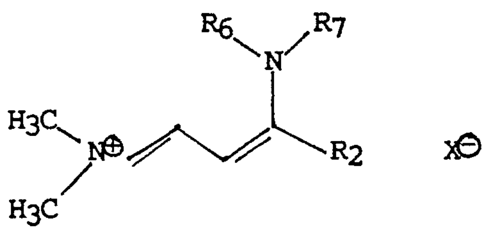

(III)

dans laquelle $R_2$, $R_6$ et $R_7$ ont les mêmes significations que précédemnent et X représente $BF_4$, Cl, $ClO_4$, Br sur un composé de formule :

$R_3$ - $CH_2$ - $R_4$

dans laquelle $R_3$ et $R_4$ ont les mêmes significations que précédemment puis isole le produit.

3 - Procédé de préparation des énamines de formule :

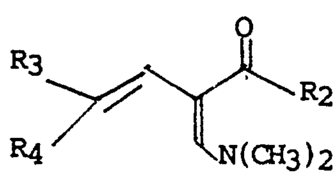

(VI)

dans laquelle $R_3$ et $R_4$ ont les mêmes significations que dans la formule (I) de la revendication 1 et $R_2$ représente un radical alcoxycarbonyle caractérisé en ce que l'on fait réagir un dialkylacétal de N,N-diméthylformamide sur un acide correspondant de formule (I) dans laquelle $R_2$ représente le radical carboxy, $R_1$ représente le radical hydroxy, $R_3$ et $R_4$ sont définis comme précédemment puis isole le produit.

Office européen des brevets

# RAPPORT DE RECHERCHE EUROPEENNE

Numero de la demande

EP 90 12 1043

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| | Néant.<br>----- | | C 07 C 323/51<br>C 07 C 229/34<br>C 07 C 69/00<br>A 61 K 31/00 |
| | | | **DOMAINES TECHNIQUES RECHERCHES (Int. Cl.5)** |
| | | | C 07 C 323/00<br>C 07 C 229/00<br>C 07 C 69/00<br>C 07 D 335/00<br>C 07 D 295/00<br>C 07 D 317/00 |

Le présent rapport a été établi pour toutes les revendications

| Lieu de la recherche | Date d'achèvement de la recherche | Examinateur |
|---|---|---|
| LA HAYE | 13-12-1990 | VAN GEYT J.J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)